(19) **Europäisches Patentamt**
European Patent Office
Office européen des brevets

(11) **EP 1 955 650 B1**

(12) **EUROPÄISCHE PATENTSCHRIFT**

(45) Veröffentlichungstag und Bekanntmachung des Hinweises auf die Patenterteilung:
**05.10.2011 Patentblatt 2011/40**

(51) Int Cl.:
**A61B 5/053** (2006.01)

(21) Anmeldenummer: **07023195.6**

(22) Anmeldetag: **30.11.2007**

(54) **Implantierbares medizinisches Gerät**

Implantable medical device

Appareil médical implantable

(84) Benannte Vertragsstaaten:
**CH DE FR GB IE LI SE**

(30) Priorität: **08.02.2007 DE 102007006229**

(43) Veröffentlichungstag der Anmeldung:
**13.08.2008 Patentblatt 2008/33**

(73) Patentinhaber: **Biotronik CRM Patent AG**
**6341 Baar (CH)**

(72) Erfinder:
- **Lippert, Michael, Dr.**
**91522 Ansbach (DE)**
- **Skerl, Olaf, Dr.**
**18209 Bad Doberan (DE)**
- **Paule, Stefan, Dr.**
**96117 Drosendorf (DE)**
- **Fahn, Bernhard, Dr.**
**90408 Nürnberg (DE)**
- **Czygan, Gerald, Dr.**
**91054 Buckenhof (DE)**
- **Reinke, Heinrich, Dr.**
**90763 Fürth (DE)**
- **Urbaszek, Albrecht, Dr.**
**91353 Hausen (DE)**

(74) Vertreter: **Lindner-Vogt, Karin L.**
**Biotronik SE & Co. KG**
**Woermannkehre 1**
**12359 Berlin (DE)**

(56) Entgegenhaltungen:
WO-A1-01/88521 US-A- 5 526 808
US-A1- 2006 025 661 US-A1- 2006 041 280

**Beschreibung**

Die Erfindung betrifft ein implantierbares medizinisches Gerät mit Mitteln zum Bestimmen von Werten, die eine Änderung eines Hämatokrit-Wertes von Blut in charakteristischer Weise widerspiegeln. Insbesondere betrifft die Erfindung implantierbare Herzschrittmacher oder implantierbare Cardioverter/Defibrillatoren oder eine Kombination aus beiden, die zum Erfassen von mit einer Änderungen des Hämatokrit-Wertes von Blut einhergehenden Werten ausgebildet sind.

Aus dem Stand der Technik, beispielsweise aus WO 2006/017446, sind Herzschrittmacher bekannt, die dazu ausgebildet sind, einen Hämatokrit-Wert auf Basis einer Blutleitfähigkeitsmessung oder Blutimpedanzmessung zu schätzen.

Das aus WO 2006/017446 bekannte Gerät nutzt die Tatsache, dass die elektrische Leitfähigkeit des Blutes in erster Linie vom jeweiligen Hämatokrit-Wert abhängt.

Der Hämatokrit-Wert (HCT) bezeichnet den Volumenanteil zellulärer Bestandteile des Blutes und ist ein Maß für die Zähflüssigkeit des Blutes.

Der Hämatokrit-Wert ist darüber hinaus eine wichtige physiologische Größe, deren Beobachtung Rückschlüsse auf den pathophysiologischen Zustand eines jeweiligen Patienten zulässt. Insbesondere sind hier Änderungen des jeweiligen Hämatokrit-Wertes von Interesse. Das Beobachten der Veränderung eines jeweiligen Hämatokrit-Wertes kann dazu genutzt werden, eine jeweilige Medikamententherapie zu optimieren.

Typische, aus dem Stand der Technik bekannte Verfahren zum Bestimmen des Hämatokrit-Wertes beruhen auf dem Zentrifugieren, Zählen der Erythrozyten, auf optischen Verfahren oder auf der Impedanzmessung.

Außerdem kann ein jeweils bekannter Hämatokrit-Wert als Korrekturwert bei der Bestimmung der Thorax-Impedanz verwendet werden, wie aus US 2006/041280 bekannt ist.

Der Erfindung liegt die Aufgabe zugrunde, ein implantierbares medizinisches Gerät zu schaffen, das möglichst zuverlässige Werte liefert, die eine Änderung des jeweiligen Hämatokrit-Wertes anzeigen. Eine Bestimmung des absoluten Hämatokrit-Wertes steht somit nicht im Vordergrund. Vielmehr interessiert die relative Änderung des Hämatokrit-Wertes. Dem liegt die Erkenntnis zugrunde, dass insbesondere die Änderung des jeweiligen Hämotokrit-Wertes von Interesse ist.

Erfindungsgemäß wird diese Aufgabe durch ein implantierbares medizinisches Gerät gelöst, das eine Impedanz- oder Admittanz-Bestimmungseinheit aufweist, die mit intrakardial oder intraluminal zu platzierenden Elektroden für die Abgabe eines Messstroms verbunden oder zu verbinden ist. Darüber hinaus weist das implantierbare medizinische Gerät eine Wechselstrom- oder Wechselspannungsquelle auf, die ausgebildet ist, den Messstrom als Wechselstrom mit konstanter Stromamplitude oder durch Wechselspannung mit konstanter Spannungsamplitude zu erzeugen und im Betrieb über an die Wechselstrom- oder Wechselspannungsquelle angeschlossene Elektroden in den Körper eines Patienten einzuspeisen. Weiterer Bestandteil des erfindungsgemäßen implantierbaren medizinischen Gerätes ist eine Messeinheit, die ausgebildet ist, im Betrieb einen infolge des eingespeisten konstanten Messstroms hervorgerufenen Spannungsabfall zwischen zwei der Elektroden oder die Stromstärke des mit konstanter Spannung eingespeisten Messstroms zu erfassen. Weiterer Bestandteil des erfindungsgemäßen implantierbaren medizinischen Gerätes ist eine Auswerteeinheit, die mit der Wechselstrom- oder Wechselspannungsquelle und der Messeinheit verbunden und ausgebildet ist, für verschiedene Zeitpunkte aus der konstanten Stromamplitude des jeweils eingespeisten Messstroms und dem infolge dessen gemessenen Spannungsabfall oder der jeweiligen konstanten Spannungsamplitude der eingespeisten Messspannung und der daraus resultierenden Stromstärke des Messstroms einen Impedanzwert oder einen Admittanzwert zu bilden. Dabei ist die Impedanz- oder Admittanzbestimmungseinheit weiterhin dazu ausgebildet, Messströme mit zwei unterschiedlichen Frequenzen unterhalb von 500 kHz und vorzugsweise unterhalb 200 kHz bzw. 100 kHz zu erzeugen. Die Auswerteeinheit ist weiterhin dazu ausgebildet, jeweils Paare von zwei einander zeitlich zugeordneten Impedanz- oder Admittanzwerten für unterschiedliche Frequenzen des Messstroms zu bilden aus einem jeweiligen Paar von Impedanzwerten einen von der Impedanz des ein jeweiliges Blutgefäß umgebenden Körpergewebes weitestgehend unabhängigen Wert für einen Blutimpedanz- oder Blutadmittanzanteil zu bilden, um dann aus für unterschiedliche Zeitpunkte bestimmten Werten des Blutimpedanz- oder Blutadmittanzanteils eine relative Änderung dieses Wertes über die Zeit als Indikator für eine Änderung eines Hämatokrit-Wertes des Blutes zu bestimmen.

Die alternativ zur Impedanz zu bestimmende Admittanz ist der Kehrwert der Impedanz und somit ein von der Frequenz eines jeweiligen Wechselstroms abhängiger Blutleitfähigkeitswert.

Die Erfindung beruht auf der Erkenntnis, dass die Bestimmung eines für eine Änderung eines jeweiligen Hämatokrit-Wertes charakteristischen Blutimpedanztrends oder Blutadmittanztrends dadurch erschwert wird, dass die Blutleitfähigkeit (Blutadmittanz) und dementsprechend auch die Blutimpedanz nicht nur vom jeweiligen Hämatokrit-Wert, sondern auch von der Bluttemperatur abhängt. Darüber hinaus spielt die jeweilige Geometrie der für die Impedanzmessung herangezogenen Elektroden sowie deren Position relativ zum Körpergewebe eine Rolle. Letztere kann sich im Laufe der Zeit ändern. Weiterhin geht in jede intraluminale oder intrakardiale Blutimpedanzmessung (also in jede intrakorporale Blutimpedanzmessung) nicht nur die jeweilige Blutimpedanz (beziehungsweise Blutadmittanz) ein, sondern auch die Impedanz beziehungsweise Admittanz des umgebenden Körpergewebes.

Dabei ist die Blutimpedanz in einem Frequenzbereich zwischen 1 kHz und 100 kHz annähernd konstant, während die Impedanz des Körpergewebes, insbesondere die Impedanz des Myokards (Muskelgewebe des Herzens), in aus der Literatur bekannter Weise von der Frequenz des jeweiligen Messstroms abhängig ist. Beispielsweise ändert sich die Leitfähigkeit und damit die Impedanz des Myokards zwischen 2 kHz und 20 kHz um den Faktor 1,48.

Da der Strompfad durch das Blut und der Strompfad durch das Gewebe als parallele Strompfade zu betrachten sind, ergibt sich, dass die Gesamtadmittanz Y(f) wenigstens annähernd durch die nachfolgende Formel zu beschreiben ist:

$$Y(f) = c \bullet [(1-a) \bullet \sigma_{Blut} + a \bullet \sigma_{Myokard}(f)]$$

wobei $\sigma_{Blut}$ die spezifische Leitfähigkeit des Blutes und $\sigma_{Myokard}$ die spezifische Leitfähigkeit des Myokards ist. f ist die Frequenz des Messstroms und c ist ein (annähernd konstanter) Faktor, der von der Elektrodengeometrie abhängt. a ist der unbekannte Anteil der Myokardimpedanz (beziehungsweise Myokardadmittanz) an der Gesamtadmittanz, der davon abhängt, wie Myokard und Blut in der Nähe der Messelektroden verteilt sind. Beispielsweise nimmt a zu, wenn sich die Messelektroden näher an eine jeweilige Herzwand heranbewegen.

Wenn die Impedanz bei zwei unterschiedlichen Frequenzen gemessen wird, kann der unbekannte Faktor a bestimmt werden und die Blutleitfähigkeit (Blutadmittanz) kann aus der Gesamtadmittanz isoliert werden, indem der Anteil der Myokardadmittanz an der Gesamtimpedanz mit Hilfe des Faktors a bestimmt wird. Dieser Faktor a ändert sich beispielsweise durch die Bewegung des Herzens, die Atmung, Lageänderungen des Patienten, aber auch langfristig durch Veränderungen der Elektrodengeometrie infolge Verrutschens oder Einwachsens in das Herzgewebe.

In Anbetracht des Vorstehenden ist die Auswerteeinheit vorzugsweise ausgebildet, jeweils zwei bei unterschiedlichen Frequenzen des Messstromes aufgenommene Admittanzwerte (die jeweils ein Paar bilden) auf Basis der vorstehenden Formel dahingehend auszuwerten, dass der Faktor a bestimmt wird. Auf diese Weise lässt sich der Trend der Änderung des Blutleitfähigkeitswertes um den Einfluss der Gewebeimpedanz bereinigen.

Das implantierbare medizinische Gerät ist vorzugsweise dazu ausgebildet, die Elektrodenkonfiguration zum Bestimmen des Blutadmittanzanteils oder des Blutimpedanzanteils von Zeit zu Zeit, beispielsweise von Messung zu Messung zu verändern. Dazu weist das implantierbare medizinische Gerät vorzugsweise entsprechende Schalter auf, wie z. B. eine Schaltmatrix in Form eines Halbleiters, die die Wechselstrom- oder Wechselspannungsquelle sowie die Messeinheit zwischen verschiedenen Elektroden bzw. den Anschlüssen für verschiedene Elektroden umschalten.

Durch Wechseln der Elektrodenkonfigurationen kann der Einfluss der Gewebeimpedanz auf die Gesamtimpedanz auch bestimmt werden, ohne dass dazu Messungen mit Messströmen unterschiedlicher Frequenz nötig sind. Insofern stellt das Wechseln der Messelektrodenkonfigurationen einen auch selbstständig zu verwirklichenden Erfindungsgedanken dar, der in Verbindung mit Messungen bei unterschiedlichen Frequenzen die Genauigkeit der Bestimmung der Blutimpedanz erhöht.

Geeignete Elektrodenkonfigurationen sind die Folgenden:

Bei einer besonders vorteilhaften quadrupolaren Impedanz- oder Admittanzmessung erfolgt die Stromeinspeisung über eine Gehäuseelektrode des Implantats und eine rechtsventrikuläre Tip-Elektrode. Die Messung der Spannung erfolgt über eine ventrikuläre Schockwendel und eine rechtsventrikuläre Ringelektrode. Die Gehäuseelektrode wird hierbei von einem elektrisch leitenden Teil eines Gehäuses des implantierbaren medizinischen Gerätes gebildet. Die Schockwendel ist eine ventrikuläre Defibrillationselektrode, wie sie an entsprechenden Elektrodenleitungen vorgesehen ist.

Neben dieser quadrupolaren Elektrodenkonfiguration kommen auch eine Reihe von tripolaren Konfigurationen in Frage, die in der folgenden Übersicht dargestellt sind. 1 benennt dabei jeweils das Elektrodenpaar, über das der Strom eingespeist wird, während U das Elektrodenpaar bezeichnet, über das die jeweilige Spannungsmessung erfolgt:

I: ventrikuläre Schockwendel - rechtsventrikuläre Tip-Elektrode,
U: ventrikuläre Schockwendel - rechtsventrikuläre Ringelektrode

I: ventrikuläre Schockwendel - rechtsventrikuläre Ringelektrode
U: ventrikuläre Schockwendel - rechtsventrikuläre Tip-Elektrode

I: ventrikuläre Schockwendel - Gehäuseelektrode,
U: ventrikuläre Schockwendel - rechtsventrikuläre Tip-Elektrode

I: ventrikuläre Schockwendel - Gehäuseelektrode,
U: ventrikuläre Schockwendel - rechtsventrikuläre Ringelektrode.

Bei diesen tripolaren Elektrodenkonfigurationen wird somit vorzugsweise die ventrikuläre Schockwendel als gemeinsame Elektrode für die Stromeinspeisung und die Spannungsmessung verwendet.

Für den Fall, dass das implantierbare medizinische Gerät nicht mit einer Elektrodenleitung zum Abgeben von ventrikulären Defibrillationsschocks verbunden ist, so dass die Elektrodenleitung keine ventrikuläre Schockwendel aufweist, kommen die folgenden Konfigurationen in Frage:

1: rechtsventrikuläre Ringelektrode - Gehäuseelektrode,
U: rechtsventrikuläre Ringelektrode - rechtsventrikuläre Tip-Elektrode

I: rechtsventrikuläre Ringelektrode - rechtsatriale Ringelektrode,
U: rechtsventrikuläre Ringelektrode - rechtsventrikuläre Tip-Elektrode,

1: rechtsatriale Ringelektrode - Gehäuseelektrode,
U: rechtsatriale Ringelektrode - rechtsatriale Tip-Elektrode

I: rechtsatriale Ringelektrode - rechtsventrikuläre Ringelektrode,
U: rechtsatriale Ringelektrode - rechtsatriale Tip-Elektrode

Die nachfolgend genannten bipolaren Elektrodenkonfigurationen haben den Nachteil, dass der Anteil der Myokardimpedanz an der gesamten Impedanz größer ist als bei den vorgenannten Elektrodenkonfigurationen, da die jeweilige Tip-Elektrode eine große Nähe zur Herzwand aufweist. Die folgenden bipolaren Konfigurationen kommen dennoch grundsätzlich ebenfalls in Frage:

I: rechtsventrikuläre Tip-Elektrode - rechtsventrikuläre Ringelektrode,
U: rechtsventrikuläre Tip-Elektrode - rechtsventrikuläre Ringelektrode

I: rechtsatriale Tip-Elektrode - rechtsatriale Ringelektrode,
U: rechtsatriale Tip-Elektrode - rechtsatriale Ringelektrode.

Konfigurationen, bei denen die Gehäuseelektrode eine gemeinsame Elektrode bildet, sind nach Möglichkeit zu vermeiden, da die Impedanz des das implantierbare medizinische Gerät umgebenden Gewebes sowie die Lungenimpedanz dann einen unnötig größeren Einfluss auf das Messergebnis haben.

Die Auswerteeinheit ist - wie weiter vorne schon angedeutet - vorzugsweise so ausgebildet, dass sie für verschiedene Zeitpunkte und jeweils zwei unterschiedliche Frequenzen des Messstroms aus der konstanten Stromstärke des jeweils eingespeisten Messstroms und dem infolge dessen gemessenen Spannungsabfall Paare von zusammengehörenden Impedanzwerten bildet. Die beiden zusammengehörenden Impedanzwerte werden dabei gleichzeitig beziehungsweise quasi gleichzeitig also unmittelbar hintereinander gebildet, so dass zwischen beiden allenfalls ein zu vernachlässigender Zeitabstand besteht. Der jeweilige Messstrom mit konstanter Stromstärke aber jeweils unterschiedlicher Frequenz wird dabei vorzugsweise in Form zweiphasiger Stromimpulse abgegeben. Der Spannungsabfall wird gemessen, so dass dann einfach die Impedanz bestimmt werden kann.

Dabei ist es bevorzugt, wenn die Auswerteeinheit für das Bestimmen der relativen Änderungen des Wertes des Blutimpedanzanteils über die Zeit (also für die Bestimmung des Trendes der Blutimpedanz) nicht alle aufgenommenen Paare zusammengehörender Impedanzwerte berücksichtigt, sondern in einem jeweiligen Zeitfenster oder innerhalb einer jeweiligen Folge von Paaren nur diejenigen Paare von Impedanzwerten berücksichtigt, die die niedrigsten Impedanzwerte repräsentieren. Beispielsweise könnte die Auswerteeinheit ausgebildet sein, für ein jeweiliges Zeitfenster oder für eine jeweilige Folge von Paaren von Impedanzwerten dasjenige Quartil zu bilden, das die niedrigsten Impedanzwerte repräsentiert. Ein Quartil enthält dabei ein Viertel der der Größe nach sortierten Paare von Impedanzwerten für ein jeweiliges Zeitfenster beziehungsweise eine jeweilige Folge. Nach Bestimmen des niedrigsten Quartils von Paaren von Impedanzwerten werden die Paare von Impedanzwerten über dieses Quartil gemittelt. Hintergrund ist, dass kurzfristige Änderungen der Impedanzwerte beispielsweise infolge der Herzbewegung oder der Atmung zur Folge haben, dass von einer Folge von Impedanzwerten die niedrigsten Impedanzwerte jeweils am stärksten auf die Blutimpedanz zurückgehen und am wenigsten von der Gewebeimpedanz beeinflusst sind, da die Impedanz des Blutes geringer ist, als die Impedanz des umgebenden Gewebes. Dementsprechend sind die niedrigsten Impedanzwerte innerhalb eines Zeitfensters oder einer Folge von Impedanzwerten diejenigen Impedanzwerte, die am wenigsten von Bewegungsartefakten beeinflusst sind.

Darüber hinaus kann es vorteilhaft sein, wenn die Auswerteeinheit ausgebildet ist, kurzzeitige Änderungen der Impedanzwerte durch Mitteln des Signals über verschiedene Zeitfenster zu eliminieren.

Wie zuvor schon erwähnt, hängt die Blutleitfähigkeit beziehungsweise der Widerstand des Blutes (die Impedanz) nicht nur vom Hämatokrit-Wert, sondern auch von der Bluttemperatur ab. Für Blut mit einem Hämatokrit-Wert im Bereich zwischen 16% und 52,5% hängt der spezifische Blutwiderstand p bei einem Messstrom mit einer Frequenz von 100 kHz innerhalb eines Temperaturbereiches zwischen 22°C und 40°C wie folgt von der Bluttemperatur und dem Hämatokrit-Wert ab:

$$\rho = [(627{,}2 \bullet HTC + 75{,}176) - (10{,}4 \bullet HTC + 1{,}467) \bullet T]$$

wobei die Temperatur T in °C einzusetzen ist und der spezifische Widerstand die Einheit $\Omega$cm hat. Um den Einfluss der Körpertemperatur und damit der Bluttemperatur bei der Bestimmung des Trendes der Blutimpedanzänderung ausblenden und so den Einfluss des Hämatokrit-Wertes isolieren zu können, weist das implantierbare medizinische Gerät vorzugsweise eine Temperaturbestimmungseinheit auf, die mit einem Temperatursensor in einer Elektrodenleitung zu verbinden und zum Bestimmen von Bluttemperaturänderungen ausgebildet ist. Die Temperaturbestimmungseinheit ist mit der Auswerteeinheit verbunden und die Auswerteeinheit ist ihrerseits ausgebildet, Änderungen der Bluttemperatur bei dem Bestimmen der relativen Änderung des Wertes der Blutimpedanz- und des Blutadmittanzanteils über die Zeit zu berücksichtigen. Der Temperatursensor ist dabei vorzugsweise in der Nähe der Elektroden untergebracht, über die beispielsweise die Messung der Spannung erfolgt.

Vorzugsweise weist das implantierbare medizinische Gerät außerdem einen Speicher für von der Auswerteeinheit bestimmte relative Blutimpedanzwerte auf. Auf diese Weise können eine Vielzahl von Blutimpedanzwerten gespeichert und der Trend von deren Änderung erfasst werden. Der Speicher ist vorzugsweise mit einer Telemetrieeinheit zum drahtlosen Übertragen von relativen Blutimpedanzwerten verbunden. Auf diese Weise können diese Blutimpedanzwerte beispielsweise zu einem zentralen Service Center übertragen und dort entsprechend gesammelt und auch über größere Zeiträume ausgewertet werden.

In diesem Zusammenhang ist es vorteilhaft, wenn die Auswerteeinheit dazu ausgebildet ist, die Amplitude einer circadianen Schwankung der gemessenen Impedanzwerte zu bestimmen. Besonders bevorzugt ist eine Auswerteeinheit, die ausgebildet ist, die Änderungen der Amplitude einer circadianen Schwankung der gemessenen Impedanzwerte als Indikatoren für Änderungen des Hämatokritwertes zu erfassen

Das implantierbare medizinische Gerät ist vorzugsweise ein implantierbarer Herzschrittmacher oder ein implantierbarer Cardioverter/Defibrillator oder eine Kombination aus beiden. Diese Geräte sind im Betrieb üblicherweise mit Elektrodenleitungen verbunden, die wiederum üblicherweise die vorgenannten Elektroden aufweisen.

Vorzugsweise ist das implantierbare medizinische Gerät dazu ausgebildet, in regelmäßigen Abständen, beispielsweise in Abständen von 24 Stunden, zwischenzeitlich gemessene Blutimpedanzwerte auf ein externes Gerät zu übertragen.

Darüber hinaus kann entweder das implantierbare medizinische Gerät, speziell dessen Auswerteeinheit, oder ein externes Gerät, das telemetrisch übertragene Blutimpedanzwerte empfängt und auswertet, so ausgebildet sein, dass das Gerät einen automatischen Alarm erzeugt, wenn sich die Blutimpedanz oder die Blutleitfähigkeit plötzlich oder schnell ändert.

Darüber hinaus kann entweder das implantierbare Gerät selbst oder ein externes Gerät, das Blutimpedanzwerte empfängt, dazu ausgebildet sein, die Schwankungen der Blutimpedanz oder Blutleitfähigkeit im Laufe eines circadianen Rhythmus zu erfassen. Ein Rückgang das Hämatokrit-Wertes ist in der Regel auch mit einem Rückgang der Schwankungsamplitude der Blutimpedanz oder des Blutleitfähigkeitswertes verbunden. Da der circadiane Rhythmus (Tagesrhythmus) eine relativ lang dauernde Periode hat, ist die Erfassung der Amplitude der Blutimpedanz oder der Blutleitfähigkeitsänderung im circadianen Rhythmus weniger anfällig für Störungen.

Weiterhin kann das implantierbare medizinische Gerät selbst oder ein externes Gerät dazu ausgebildet sein, aus einer auf eine Änderung des Hämatokrit-Wertes zurückgehende Änderung der Blutimpedanz Korrekturwerte für andere Impedanzmessungen, beispielsweise für die Messung der Lungenimpedanz oder für die Impedanz-Plethysmographie zu liefern. Beispielsweise werden Impedanzmessungen durchgeführt, um die Änderungen der Herzgeometrie oder der Lungengeometrie oder der Leitfähigkeit des Lungengewebes zu erfassen. Auch werden Impedanzmessungen per Impedanz-Plethysmographie zur Bestimmung der Volumenänderungen des linken Ventrikels, oder zur Bestimmung des Flüssigkeitsgehaltes der Lunge oder zum Bestimmen der Respirationsrate oder zum Bestimmen des Atemvolumens oder zum Bestimmen der rechtsventrikulären apikalen Kontraktionsdynamik herangezogen. All diese Messungen können hinsichtlich einer Änderung der Blutleitfähigkeit infolge eines sich ändernden Hämatokrit-Wertes korrigiert werden, wenn eine Änderung des Hämatokrit-Wertes mit einem implantierbaren medizinischen Gerät der erfindungsgemäßen Art erfasst wird.

Eine weitere Anwendung des erfindungsgemäßen implantierbaren medizinischen Gerätes besteht im Erfassen eines Thrombus im rechten oder linken Atrium in Folge Vorhofflimmerns. Die Bildung eines Thrombus ändert die lokale Leitfähigkeit des Blutes und kann ebenfalls durch Auswerten der Blutimpedanz im Atrium erfasst werden. Hier kommt insbesondere eine Impedanzmessung über atriale Elektroden in Frage, beispielsweise sowohl Stromeinspeisung als auch Spannungsmessung über die rechte atriale Tip-Elektrode und die rechte atriale Ringelektrode.

Es versteht sich, dass die verschiedenen hier beschriebenen und mit den Unteransprüchen beanspruchten Aspekte - soweit sie sich nicht gegenseitig ausschließen - miteinander kombiniert werden können.

Die Erfindung soll nun anhand von Ausführungsbeispielen mit Bezug auf die Figuren näher erläutert werden.

Von den Figuren zeigen:

Fig. 1:     einen implantierbaren Herzschrittmacher/Defibrillator mit daran angeschlossenen Elektrodenleitungen und deren Platzierung im Herzen eines Menschen;

Fig. 2:     ein schematisches Blockschaltbild einer ersten Variante eines erfindungsgemäßen Herzschrittmachers/Defibrillators; und

Fig. 3:     ein vereinfachtes Blockschaltbild einer zweiten Variante des erfindungsgemäßen Herzschrittmachers/Defibrillators.

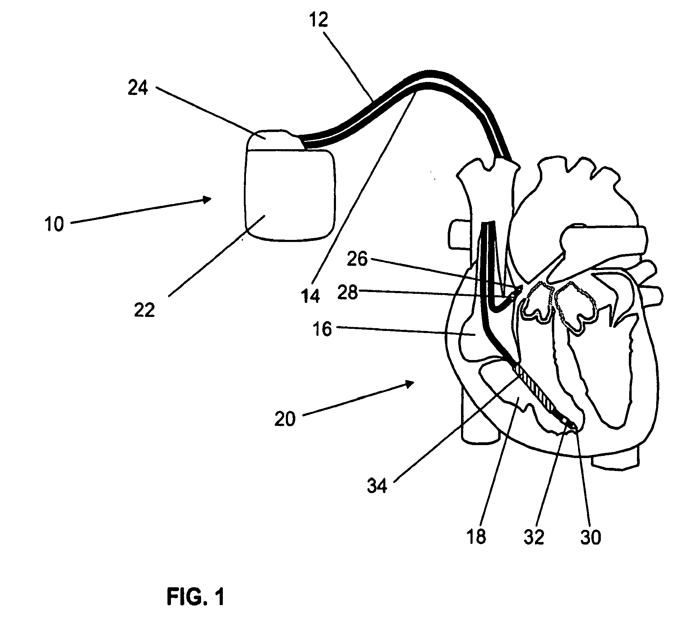

Figur 1 zeigt, wie ein implantierbares medizinisches Gerät 10, in diesem Falle ein Herzschrittmacher/Defibrillator über Elektrodenleitungen 12 und 14 mit verschiedenen Elektroden verbunden ist, die in einem Atrium 16 und einem Ventrikel 18 eines Herzens 20 angeordnet sind.

Die Elektrodenleitungen 12 und 14 sind an ihrem jeweiligen proximalen Ende mittels eines Steckkontaktes mit einer Elektronik im Inneren eines Gehäuses 22 des Herzschrittmachers 10 verbunden. Das Gehäuse 22 des Herzschrittmachers 10 ist hermetisch dicht und besteht aus einem leitfähigen, biokompatiblen Werkstoff, beispielsweise Titan. Am Gehäuse 22 ist ein sogenannter Header 24 befestigt, der typischerweise aus transparentem Kunststoff besteht und Buchsen für die Aufnahme der Stecker an dem jeweiligen proximalen Ende der Elektrodenleitungen 12 und 14 aufweist. Diese Buchsen weisen nicht weiter dargestellte elektrische Kontakte auf, die über eine Durchführung mit der Elektronik im Inneren des Gehäuses 22 des Herzschrittmachers 10 elektrisch verbunden sind.

Von den beiden Elektrodenleitungen 12 und 14 ist die eine Elektrodenleitung eine rechtsatriale Elektrodenleitung 12. Sie trägt an ihrem distalen Ende eine rechtsatriale Tip-Elektrode 26 und in geringer Entfernung davon eine rechtsatriale Ringelektrode 28. Die rechtsatriale Tip-Elektrode 26 und die rechtsatriale Ringelektrode 28 befinden sich im implantierten Zustand der atrialen Elektrodenleitung 12 im Atrium 16 des Herzens 20.

Die andere Elektrodenleitung ist eine rechtsventrikuläre Elektrodenleitung 14. Diese trägt an ihrem distalen Ende eine rechtsventrikuläre Tip-Elektrode 30 und in geringer Entfernung davon eine rechtsventrikuläre Ringelektrode 32. Außerdem ist auf der rechtsventrikulären Elektrodenleitung 14 eine ventrikuläre Schockwendel 34 angeordnet, die eine großflächige Elektrode zur Abgabe von Defibrillationsschocks bildet und der Beweglichkeit halber üblicherweise helixförmig gewendelt ausgebildet ist.

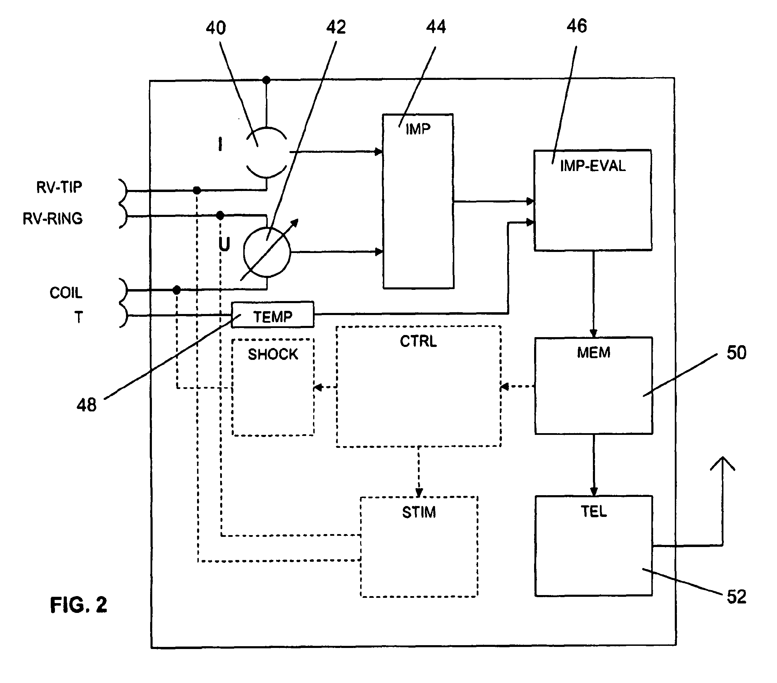

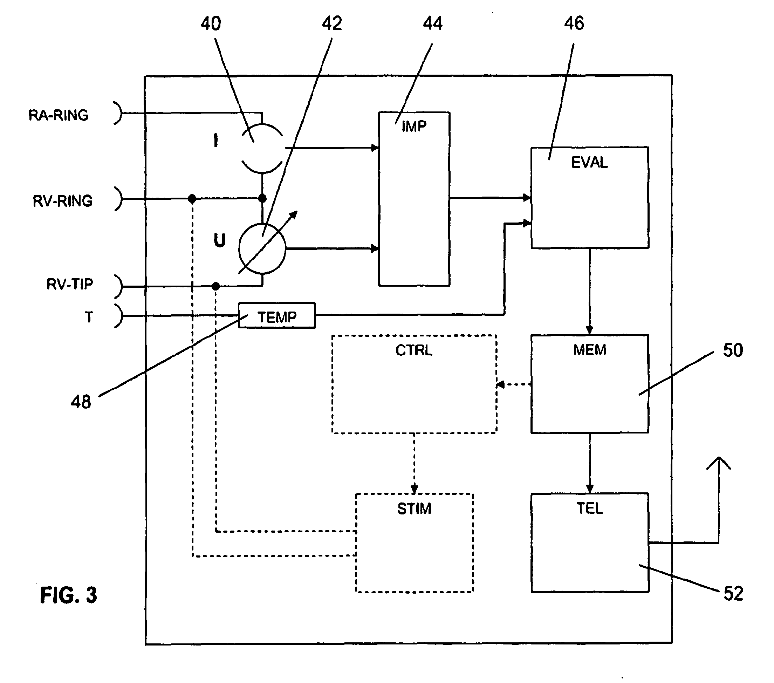

Figuren 2 und 3 zeigen beispielhaft einige der im Inneren des Gehäuses 22 des Herzschrittmachers beziehungsweise Defibrillators 10 angeordneten Bestandteile in schematischer Darstellungsweise. Nicht dargestellt, beziehungsweise teilweise nur angedeutet sind die typischen Bestandteile eines implantierbaren Herzschrittmachers oder Defibrillators wie atriale und ventrikuläre Sensingstufen, Stimulationsimpulsgeneratoren, Defibrillationsschockgeneratoren, eine Steuereinheit etc. Diese Bestandteile und ihre Varianten sind dem Fachmann wohlbekannt.

Im Zusammenhang mit der vorliegenden Erfindung besonders relevant sind eine Wechselstromquelle 40, eine Spannungsmesseinheit 42, eine Impedanzbestimmungseinheit 44 und eine Impedanzauswerteeinheit 46. Die Wechselstromquelle 40 ist dazu ausgebildet, einen Messstrom in Form biphasischer Stromimpulse mit konstanter Stromstärke zu erzeugen. Der von der Wechselstromquelle 40 erzeugte Messstrom kann jeweils eine von zwei Wechselstromfrequenzen unterhalb 100 kHz haben.

Die Wechselstromquelle 40 ist in der in Figur 2 dargestellten Konfiguration einerseits mit dem elektrisch leitenden Gehäuse 22 des Herzschrittmachers 10 und andererseits mit dem Anschluss RV-Tip für eine rechtsventrikuläre Tip-Elektrode elektrisch leitend verbunden.

Die Messeinheit 42 ist in der in Figur 2 abgebildeten Konfiguration einerseits mit dem Anschluss RV-Ring für eine rechtsventrikuläre Ringelektrode und dem Anschluss COIL für eine Schockwendel verbunden und dazu ausgebildet, die zwischen diesen beiden Anschlüssen infolge der Abgabe des Messstroms durch die Wechselstromquelle 40 hervorgerufene Wechselspannung zu messen.

Bei dem abgebildeten Herzschrittmacher 10 sind die Ausgänge der Wechselstromquelle 40 und die Eingänge der Messeinheit 42 umschaltbar und auf diese Weise mit anderen zur Verfügung stehenden Anschlüssen verbindbar. Hieraus ergeben sich verschiedene mögliche Elektrodenkonfigurationen für die Impedanz- oder Admittanzmessung.

Dies ist durch Figur 3 angedeutet. Dort ist die Wechselstromquelle 40 einerseits mit einem Anschluss für eine rechtsatriale Ringelektrode und andererseits mit dem Anschluss für die rechtsventrikuläre Ringelektrode verbunden, während die Messeinheit 42 mit dem Anschluss für die rechtsventrikuläre Ringelektrode RV-Ring und dem Anschluss RV-Tip für die rechtsventrikuläre Tip-Elektrode verbunden ist.

Ein Schalter, der das unterschiedliche Verbinden der Ausgänge der Wechselstromquelle 40 und der Eingänge der Messeinheit 42 bewirkt, ist in den Figuren nicht dargestellt. Grundsätzlich kann aber die Umschaltbarkeit der Anschlüsse der Wechselstromquelle und der Messeinheit zwischen allen in Figur 1 dargestellten Elektroden vorgesehen sein. Im Falle biventrikulärer Implantate oder atrialer Defibrillatoren kämen noch weitere Elektroden hinzu.

Die Wechselstromquelle 40 und die Messeinheit 42 sind mit der Impedanzbestimmungseinheit 44 verbunden, die aus einem jeweils abgegebenen Messstrom und der zugehörigen, von der Messeinheit 42 erfassten Spannung einen Impedanzwert bildet.

Die Impedanzbestimmungseinheit 44 ist mit der Impedanzauswertungseinheit 46 verbunden. Beide zusammen bilden eine Auswerteeinheit im Sinne der Ansprüche.

Die Impedanzauswertungseinheit 46 ist dazu ausgebildet, jeweils Paare von Impedanzwerten zu bilden, wobei die zu Paaren zusammengefassten Impedanzwerte jeweils zu annähernd gleichem Zeitpunkt und mit jeweils einem Messstrom unterschiedlicher Frequenz gewonnen werden. Die Impedanzauswertungseinheit 46 ist weiterhin ausgebildet, aus diesen Paaren von (Gesamt-) Impedanzwerten einen Blutimpedanzanteil zu bilden, in dem die Impedanzauswertungseinheit 46 einen Gewebeimpedanzanteil auf die weiter vorne beschriebene Art und Weise eliminiert.

Ebenso ist die Impedanzauswerteeinheit 46 dazu ausgebildet, ein gemitteltes Impedanzwertpaar über ein Quartil aller niedrigsten Impedanzwerte innerhalb eines vorgegebenen Zeitraums oder einer vorgegebenen Folge von Impedanzwerten zu bilden.

Schließlich ist die Impedanzauswertungseinheit 46 mit einer Temperaturbestimmungseinheit 48 verbunden, die ihrerseits mit einem in der Nähe der Elektroden für die Spannungsmessung angeordneten Temperatursensor verbunden ist. Auf diese Weise ist die Impedanzauswerteeinheit 46 in der Lage, Änderungen der Bluttemperatur bei der Auswertung der Impedanzwerte zu berücksichtigen und den auf eine Änderung des Hämatokrit-Wertes zurückgehenden Anteil an einer Änderung der Blutimpedanz zu isolieren.

Die Impedanzauswertungseinheit liefert im Ergebnis einen Ausgangswert, der derart mit der Blutimpedanz korreliert ist, dass Änderungen des Ausgangswertes der Impedanzauswertungseinheit eine Änderung der Blutimpedanz infolge einer Änderung des Hämatokrit-Wertes widerspiegeln, die weitestgehend frei ist von zunächst mitgemessenen Gewebeimpedanzen, Blutimpedanzänderungen infolge von Temperaturänderungen oder Blutimpedanzänderungen infolge von Elektrodenbewegungen oder Bewegungen der die Elektroden umgebenden Gefäße. Der Ausgangswert der Impedanzauswertungseinheit hängt somit vor allem vom Hämatokrit-Wert des Blutes ab, so dass Änderungen des Ausgangswertes der Impedanzauswertungseinheit 46 mit Änderungen des Hämatokrit-Wertes des Blutes einhergehen und diese widerspiegeln. Daher ist der Ausgangswert der Impedanzauswertungseinheit 46 nützlich im Zusammenhang mit der Beobachtung einer Änderung des Hämatokrit-Wertes des Blutes eines Patienten.

Die Impedanzbestimmungseinheit 44 und die Impedanzauswertungseinheit 46 bilden zusammen eine Auswerteeinheit, die den vorbeschriebenen Ausgangswert liefert.

Dieser Ausgangswert der Auswerteeinheit, dessen Änderungen letztendlich Änderungen des Hämatokrit-Wertes des Blutes eine Patienten widerspiegeln, wird einem Speicher 50 zugeführt, der seinerseits mit einer Telemetrieeinheit 52 verbunden ist. auf diese Weise können die Ausgangswerte der Impedanzauswerteeinheit 46 zum einen über einen längeren Zeitraum für die Trendbestimmung gespeichert und zum anderen mittels der Telemetrieeinheit 52 an ein externes Gerät zur weiteren Auswertung übertragen werden.

In Figuren 2 und 3 nur angedeutet sind weitere Bestandteile des Herzschrittmachers/Defibrillators 10 wie beispielsweise eine Steuereinheit, ein Schockimpulsgenerator oder ein Stimulationsimpulsgenerator.

## Patentansprüche

**1.** Implantierbares medizinisches Gerät (10) mit

einer Impedanz- oder Admittanzbestimmungseinheit (40, 42, 44, 46), die mit intrakardial oder intraluminal zu platzierenden Elektroden für die Abgabe eines Messstroms verbunden oder zu verbinden ist und

einer Wechselstrom- oder Wechselspannungsquelle (40), die ausgebildet ist, einen Messstrom als Wechselstrom konstanter Stromamplitude oder durch Wechselspannung konstanter Spannungsamplitude zu erzeugen und im Betrieb den Messstrom bei angeschlossenen Elektroden in den Körper eines Patienten einzuspeisen,

einer Messeinheit (42), die ausgebildet ist, im Betrieb einen infolge des eingespeisten konstanten Messstroms hervorgerufenen Spannungsabfall zwischen zwei der Elektroden oder die Stromstärke des mit konstanter Spannung eingespeisten Messstroms zu erfassen, sowie

einer Auswerteeinheit (44, 46), die mit der Wechselstrom- oder Wechselspannungsquelle (40) und der Messeinheit (42) verbunden und ausgebildet ist, für verschiedene Zeitpunkte aus der konstanten Stromamplitude des jeweils eingespeisten Messstroms und des infolgedessen gemessenen Spannungsabfalls oder aus der jeweiligen konstanten Spannungsamplitude der eingespeisten Messspannung und der daraus resultierenden Stromstärke des Messstroms einen Impedanzwert oder einen Admittanzwert zu bilden,

**dadurch gekennzeichnet, dass**

die Impedanz- oder Admittanzbestimmungseinheit (40, 42, 44, 46) weiterhin ausgebildet ist, Messströme mit zwei unterschiedlichen Frequenzen unterhalb von 500 kHz zu erzeugen und

die Auswerteeinheit (44, 46) weiterhin ausgebildet ist,

jeweils Paare von einander zeitlich zugeordneten Impedanz- oder Admittanzwerten für unterschiedliche Frequenzen des Messstroms zu bilden und aus einem jeweiligen Paar von Impedanzwerten einen von der Impedanz des ein jeweiliges Blutgefäß umgebenden Körpergewebes unabhängigen Wert für einen Blutimpedanz- oder Blutadmittanzanteil zu bilden und

aus für unterschiedliche Zeitpunkte bestimmten Werten des Blutimpedanz- oder Blutadmittanzanteils eine relative Änderung des Wertes des Blutimpedanz- oder Blutadmittanzanteils über die Zeit (Trend) als Indikator für eine Änderung eines Hämatokrit-Wertes des Blutes zu bestimmen.

2. Implantierbares medizinisches Gerät nach Anspruch 1, **dadurch gekennzeichnet, dass** die Auswerteeinheit (44, 46) ausgebildet ist, aus zwei Werten für die Gesamtadmittanz Y(f) als Summe von Blutadmittanz und Gewebeadmittanz für zwei unterschiedliche Frequenzen in folgender Gleichung:

$$Y(f) = c \cdot [(1-a) \cdot \sigma_{Blut} + a \cdot \sigma_{Myocard}(f)]$$

mit $\sigma_{Blut}$ und $\sigma_{Myocard}$ als den spezifischen Leitfähigkeiten von Blut und Körpergewebe den Faktor a als Anteil der Gewebeadmittanz an der Gesamtadmittanz zu bestimmen und damit Änderungen der Blutleitfähigkeit von Änderungen der Gewebeleitfähigkeit zu separieren.

3. Implantierbares medizinisches Gerät nach Anspruch 1 oder 2, **dadurch gekennzeichnet, dass** das medizinische Gerät ausgebildet ist, den Impedanz- oder Admittanzwert mit von Zeit zu Zeit wechselnden Elektrodenkonfigurationen zu messen.

4. Implantierbares medizinisches Gerät nach einem der Ansprüche 1 bis 3, **dadurch gekennzeichnet, dass** die Auswerteeinheit (44, 46) weiterhin ausgebildet ist, für verschiedene Zeitpunkte und jeweils zwei unterschiedliche Frequenzen des Messstroms aus der konstanten Stromstärke des jeweils eingespeisten Messstroms und dem infolgedessen gemessenen Spannungsabfall Paare von zusammengehörenden Impedanzwerten zu bilden.

5. Implantierbares medizinisches Gerät nach Anspruch 4, **dadurch gekennzeichnet, dass** die Auswerteeinheit (44, 46) weiterhin ausgebildet ist, für die Bestimmung der relativen Änderung des Wertes des Blutimpedanzanteils über die Zeit nur solche Paare von zusammengehörenden Impedanzwerten zu berücksichtigen, die innerhalb einer vorgegeben Anzahl von Paaren von zusammengehörenden Impedanzwerten oder innerhalb eines vorgegebenen Zeitfensters einen oder mehrere der niedrigsten Impedanzwerte repräsentieren.

6. Implantierbares medizinisches Gerät nach einem der Ansprüche 1 bis 5, **dadurch gekennzeichnet, dass** das medizinische Gerät eine Temperaturbestimmungseinheit (48) umfasst, die mit einem Temperatursensor in einer Elektrodenleitung zu verbinden und zum Bestimmen von Bluttemperaturänderungen ausgebildet ist, wobei die Temperaturbestimmungseinheit (48) mit der Auswerteeinheit (44, 46) verbunden und die Auswerteeinheit (44, 46) ausgebildet ist, Änderungen der Bluttemperatur bei dem Bestimmen der relativen Änderung des Wertes des Blutimpedanz- oder Blutadmittanzanteils über die Zeit zu berücksichtigen.

7. Implantierbares medizinisches Gerät nach einem der Ansprüche 1 bis 6, **dadurch gekennzeichnet, dass** die Wechselstromquelle (40) ausgebildet ist, einen Messstrom als Wechselstrom konstanter Stromstärke in Form biphasischer Stromimpulse zu erzeugen.

8. Implantierbares medizinisches Gerät nach einem der Ansprüche 1 bis 7, **dadurch gekennzeichnet, dass** das medizinische Gerät einen Speicher (50) für von der Auswerteeinheit bestimmte relative Blutimpedanzwerte aufweist.

9. Implantierbares medizinisches Gerät nach einem der Ansprüche 1 bis 8, **dadurch gekennzeichnet, dass** das medizinische Gerät eine Telemetrieeinheit (52) zum drahtlosen Übertragen von der Auswerteeinheit bestimmten relativen Blutimpedanzwerten aufweist.

10. Implantierbares medizinisches Gerät nach einem der Ansprüche 1 bis 9, **dadurch gekennzeichnet, dass** das medizinische Gerät ein implantierbarer Herzschrittmacher oder ein implantierbarer Cardioverter/Defibrillator oder eine Kombination aus beiden ist.

11. Implantierbares medizinisches Gerät nach einem der Ansprüche 1 bis 10, **dadurch gekennzeichnet, dass** die Auswerteeinheit (44, 46) weiterhin ausgebildet ist, die Amplitude einer circadianen Schwankung der gemessenen Impedanzwerte zu bestimmen.

12. Implantierbares medizinisches Gerät nach einem der Ansprüche 1 bis 10, **dadurch gekennzeichnet, dass** die Auswerteeinheit (44, 46) weiterhin ausgebildet ist, Änderungen der Amplitude einer circadianen Schwankung der gemessenen Impedanzwerte als Indikatoren für Änderungen des Hämatokritwertes zu erfassen.

**Claims**

1. An implantable medical device (10) comprising
   an impedance or admittance determination unit (40, 42, 44, 46), which is connected or will be connected to electrodes to be placed intracardially or intraluminally for delivering a measuring current and
   an alternating current or alternating current voltage source (40), which is embodied so as to generate a measuring current as an alternating current of constant current amplitude or using alternating current voltage at a constant voltage amplitude, and, during operation, with the electrodes connected, to deliver the measuring current to the body of a patient,
   a measuring unit (42), which is embodied to detect, during operation, a voltage drop between two of the electrodes resulting from the delivered constant measuring current, or to detect the current intensity of the measuring current delivered at a constant voltage, and
   an evaluation unit (44, 46), which is connected to the source of alternating current or alternating current voltage (40) and the measuring unit (42), and is embodied to generate an impedance value or an admittance value, for different times, from the constant current amplitude of the particular delivered measuring current and the drop in voltage measured as a result thereof, or from the constant voltage amplitude of the delivered measuring voltage and the resulting current intensity of the measuring current,
   **characterized in that** the impedance or admittance determination unit (40, 42, 44, 46) is further embodied to generate measuring currents having two different frequencies below 500 kHz, and
   the evaluation unit (44, 46) is further embodied
   to generate pairs of impedance or admittance values, assigned to one another in terms of time, for different frequencies of the measuring current, and to generate a value for a blood impedance or blood admittance component, which is independent of the impedance of the body tissue surrounding a particular blood vessel, from a respective pair of impedance values, and
   to determine, from values for the blood impedance or blood admittance component, determined for different times, a relative change in the value of the blood impedance or
   blood admittance component over time (trend) as an indicator of a change in a hematocrit value of the blood.

2. The implantable medical device according to claim 1, **characterized in that** the evaluation unit (44, 46) is embodied to calculate the factor a as a ratio of tissue admittance to total admittance using the following equation:

$$Y(f) = c \cdot [(1\text{-}a) \cdot \sigma_{blood} + \alpha \cdot \sigma_{myocardium}(f)]$$

in which $\sigma_{blood}$ and $\sigma_{myocardium}$ are the specific conductivities of blood and body tissue, from two values for total admittance Y(f), as the sum of blood admittance and tissue admittance, for two different frequencies, and thereby separate changes in blood conductivity from changes in tissue conductivity.

3. The implantable medical device according to claim 1 or 2, **characterized in that** the medical device is embodied to measure the impedance or admittance value using electrode configurations that change over time.

4. The implantable medical device according to any one of claims 1 to 3, **characterized in that** the evaluation unit (44, 46) is further embodied so as to generate pairs of related impedance values from the constant current intensity of the delivered measuring current and from the voltage drop measured as a result thereof, for different times and in each case two different frequencies of the measuring current.

5. The implantable medical device according to claim 4, **characterized in that** the evaluation unit (44, 46) is further embodied to consider only those pairs of related impedance values which represent one or more of the lowest impedance values within a predefined number of pairs of related impedance values or within a predefined window of time, in its determination of the relative change in the value of the blood impedance component over time.

6. The implantable medical device according to any one of claims 1 to 5, **characterized in that** the medical device comprises a temperature determination unit (48), which is embodied for connection to a temperature sensor in an electrode lead and for determining changes in blood temperature, wherein the temperature determination unit (48) is connected to the evaluation unit (44, 46) and the evaluation unit (44, 46) is embodied to consider changes in the blood temperature in its determination of the relative change in the value of the blood impedance or blood admittance component over time.

7. The implantable medical device according to any one of claims 1 to 6, **characterized in that** the alternating current source (40) is embodied to generate a measuring current as an alternating current of constant current intensity in the form of biphasic current pulses.

8. The implantable medical device according to any one of claims 1 to 7, **characterized in that** the medical device has a storage device (50) for relative blood impedance values determined by the evaluation unit.

9. The implantable medical device according to any one of claims 1 to 8, **characterized in that** the medical device has a telemetry unit (52) for wirelessly transmitting relative blood impedance values determined by the evaluation unit.

10. The implantable medical device according to any one of claims 1 to 9, **characterized in that** the medical device is an implantable cardiac pacemaker or an implantable cardioverterdefibrillator, or a combination thereof.

11. The implantable medical device according to any one of claims 1 to 10, **characterized in that** the evaluation unit (44, 46) is further embodied to determine the amplitude of a circadian fluctuation in the measured impedance values.

12. The implantable medical device according to any one of claims 1 to 10, **characterized in that** the evaluation unit (44, 46) is further embodied to detect changes in the amplitude of a circadian fluctuation in the measured impedance values as indicators of changes in the hematocrit value.

**Revendications**

1. Dispositif médical implantable (10) avec

   - une unité de détermination d'impédance ou d'admittance (40, 42, 44, 46), reliée ou à relier à des électrodes disposées de façon intracardiaque ou intraluminale, pour délivrer un courant de mesure, et
   - une source de tension alternative ou de courant alternatif (40), conçue pour produire un courant de mesure en tant que courant alternatif avec une amplitude de courant constante, ou par une tension alternative à amplitude de tension constante, et pour alimenter le courant de mesure dans le corps d'un patient pendant le fonctionnement, lorsque les électrodes sont connectées,
   - une unité de mesure (42) conçue pour détecter, pendant le fonctionnement, une chute de tension entre deux des électrodes, générée suite au courant de mesure constant alimenté, ou pour détecter la puissance de courant du courant de mesure alimenté avec une tension constante, et
   - une unité d'évaluation (44, 46) reliée à la source de tension alternative ou de courant alternatif (40) et à l'unité de mesure (42), et conçue pour former une valeur d'impédance ou une valeur d'admittance, pour différents moments, à partir de l'amplitude de courant constante du courant de mesure respectivement alimenté et de la chute de tension mesurée à la suite de celui-ci, ou à partir de l'amplitude de tension constante respective de la tension de mesure alimentée et de la puissance de courant résultante du courant de mesure,

   **caractérisée en ce que**

- l'unité de détermination d'impédance ou d'admittance (40, 42, 44, 46) est en outre conçue pour produire des courants de mesure avec deux fréquences différentes en-dessous de 500 kHz, et
- l'unité d'évaluation (44, 46) est en outre conçue pour
- former des paires respectives de valeurs d'impédance ou d'admittance attribuées les unes aux autres dans le temps, pour différentes fréquences du courant de mesure, et pour former une valeur pour une part d'impédance sanguine ou l'admittance sanguine, indépendante de l'impédance du tissu corporel entourant un vaisseau sanguin respectif, à partir d'une paire respective de valeurs d'impédance, et
- pour déterminer un changement relatif de la valeur de la part d'impédance sanguine ou d'admittance sanguine sur la durée (tendance), en tant qu'indicateur pour une modification de la valeur de l'hématocrite dans le sang, à partir de valeurs de part d'impédance sanguine ou d'admittance sanguine, déterminées pour différents moments.

2. Dispositif médical implantable selon la revendication 1, **caractérisé en ce que** l'unité d'évaluation (44, 46) est conçue pour déterminer le facteur a comme part de l'admittance tissulaire dans l'admittance globale, à partir de deux valeurs, pour l'admittance globale Y(f) en tant que somme de l'admittance sanguine et de l'admittance tissulaire, pour deux fréquences distinctes, dans l'équation suivante :

$$Y(f) = c \cdot [(1-a) \cdot \sigma_{Sang} + a \cdot \sigma_{Myocarde}(f)]$$

avec $\sigma_{Sang}$ et $\sigma_{Myocarde}$ en tant conductibilité spécifique du sang et des tissus corporels, et de distinguer ainsi les variations de conductibilité du sang d'avec les variations de conductibilité des tissus.

3. Dispositif médical implantable selon la revendication 1 ou 2, **caractérisé en ce que** le dispositif médical est conçu pour mesurer la valeur d'impédance ou d'admittance avec des configurations d'électrodes variant de temps à autre.

4. Dispositif médical implantable selon l'une des revendications 1 à 3, **caractérisé en ce que** l'unité d'évaluation (44, 46) est en outre conçue pour former des paires de valeurs d'impédance correspondantes, pour différents moments et à chaque fois pour deux fréquences différentes du courant de mesure, à partir de la puissance de courant constante du courant de mesure respectivement alimenté et de la chute de tension mesurée suite à celui-ci.

5. Dispositif médical implantable selon la revendication 4, **caractérisé en ce que** l'unité d'évaluation (44, 46) est en outre conçue pour ne tenir compte, dans la détermination de la variation relative de la valeur de la part d'impédance sanguine sur le temps, que les paires de valeurs d'impédance correspondantes représentant l'une ou plusieurs des valeurs d'impédance les plus faibles, parmi d'un nombre prédéfini de paires de valeurs d'impédance correspondantes, ou en l'espace d'une période de temps prédéfinie.

6. Dispositif médical implantable selon l'une des revendications 1 à 5, **caractérisé en ce que** le dispositif médical comprend une unité de détermination de la température (48), destinée à être reliée à un capteur de température dans une ligne d'électrode et conçue pour déterminer les variations de température du sang, dans lequel l'unité de détermination de la température (48) est reliée à l'unité d'évaluation (44, 46), et l'unité d'évaluation (44, 46) est conçue pour prendre en compte les variations de la température sanguine lors de la détermination de la variation relative de la valeur de la part d'impédance sanguine ou d'admittance sanguine sur la durée.

7. Dispositif médical implantable selon l'une des revendications 1 à 6, **caractérisé en ce que** la source de courant alternatif (40) est conçue pour produire un courant de mesure en tant que courant alternatif avec une puissance de courant constante, sous la forme d'impulsions de courant biphasées.

8. Dispositif médical implantable selon l'une des revendications 1 à 7, **caractérisé en ce que** le dispositif médical comporte une mémoire (50) pour les valeurs d'impédance sanguine relatives, déterminées par l'unité d'évaluation.

9. Dispositif médical implantable selon l'une des revendications 1 à 8, **caractérisé en ce que** le dispositif médical comporte une unité de télémétrie (52) pour la transmission sans fil de valeurs d'impédance sanguine relatives déterminées par l'unité d'évaluation.

10. Dispositif médical implantable selon l'une des revendications 1 à 9, **caractérisé en ce que** le dispositif médical est un stimulateur cardiaque implantable ou un cardioverter/défibrillateur implantable, ou encore une combinaison des deux.

11. Dispositif médical implantable selon l'une des revendications 1 à 10, **caractérisé en ce que** l'unité d'évaluation (44, 46) est en outre conçue pour déterminer l'amplitude d'une variation circadienne des valeurs d'impédance mesurées.

12. Dispositif médical implantable selon l'une des revendications 1 à 10, **caractérisé en ce que** l'unité d'évaluation (44, 46) est en outre conçue pour détecter les changements d'amplitude d'une variation circadienne des valeurs d'impédance mesurées, comme indicateurs pour les changements de valeur de l'hématocrite.

FIG. 1

FIG. 2

EP 1 955 650 B1

FIG. 3

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2006017446 A

- US 20060041280 A